# EUROPÄISCHE PATENTANMELDUNG

(11) **EP 1 829 553 A1**
(43) Veröffentlichungstag der Anmeldung: **05.09.2007**
(21) Anmeldenummer: 06003958.3
(22) Anmeldetag: 27.02.2006
(51) Int. Cl.: A61K 45/06

(54) **Vitamine in Omega Ölmischungen**

(71) Anmelder: EnergyBalance AG, 9491 Ruggell (LI)
(72) Erfinder:

(57) **Zusammenfassung**

Studien zeigen, dass Vitamine und Antioxidantien wie z.B. Vitamin C, auch bezeichnet als Ascorbinsäure, für die Gesundheit von Lebewesen eigentlich sehr wichtig sind. In der handelsüblichen synthetischen und isolierten chemischen Form haben sie jedoch eine schlechte Bioverfügbarkeit und verminderte Wirkung. Zudem sind wasserlösliche Vitamine, wie z.B. Ascorbinsäure, aufgrund ihrer Wasserlöslichkeit schwierig in Öle zu mischen. Das Ziel dieser Erfindung ist ölige Mischungen mit höherer Bioverfügbarkeit und besserer Wirkung herzustellen.

## Beschreibung

Studien zeigen, dass Nährstoffe wie z.B. Vitamin C, auch bezeichnet als Ascorbinsäure, für die Gesundheit von Lebewesen eigentlich sehr wichtig sind, solange sie in der natürlichen Form mit Nahrung aufgenommen werden.
Handelsübliche synthetische und isolierte chemische Formen haben eine schlechte Bioverfügbarkeit.
Anstelle den Nährstoff zu isolieren, wie das normalerweise für pharmazeutische Anwendungen üblich ist, sollte ein Nährstoff in eine biologische Struktur gebunden sein. Solche Strukturen können Nahrungs- oder Pflanzenextrakte sein, welche wie eine Matrix den Nährstoff einbinden. Die Matrix soll eine natürliche Trägerstruktur darstellen, welche die biologischen Eigenschaften für eine Aufnahme und Wirkungsentfaftung so enthält, dass sie für den Konsumenten physiologisch optimal sind. Die Matrix soll in ihrem Referenzgewicht den Nährstoff in einer klar definierten und gleichbleibenden Menge enthalten um genaue Dosierungen zu ermöglichen.
Nahrungs- bzw. strukturgebundene Nährstoffe, welche mit Omega-3 und -6 Fettsäuren gemischt werden, haben eine höhere Biowirksamkeit.

## Patentansprüche

1. Mischungen mit wasserlöslichen Vitaminen und Omega-3 und -6 Fettsäuren, welche in jeder Form medizinische, therapeutische, präventive, nahrungsergänzende und kosmetische Anwendung bei Menschen findet,
**dadurch gekennzeichnet,**
**dass** die Vitamine nicht isoliert, sondern in einem speziellen Pflanzenextrakt enthalten sind, welche die Biowirksamkeit der Vitamine erhöht.

2. Mischungen mit fettlöslichen Vitaminen und Omega-3 Fettsäuren aus Fischöl, welche in jeder Form medizinische, therapeutische, präventive, nahrungsergänzende und kosmetische Anwendung bei Menschen findet,
**dadurch gekennzeichnet,**
**dass** die Vitamine nicht isoliert, sondern in einem speziellen Pflanzenextrakt enthalten sind, welche die Biowirksamkeit der Vitamine erhöht.

3. Mischungen mit fettlöslichen Vitaminen und Omega-3 und -6 Fettsäuren, welche in jeder Form medizinische, therapeutische, präventive, nahrungsergänzende und kosmetische Anwendung bei Menschen findet,
**dadurch gekennzeichnet,**
**dass** die Vitamine nicht isoliert, sondern in einem speziellen Pflanzenextrakt enthalten sind, welche die Biowirksamkeit der Vitamine erhöht.

4. Mischungen mit Flavonoiden und Omega-3 und -6 Fettsäuren, welche in jeder Form medizinische, therapeutische, präventive, nahrungsergänzende und kosmetische Anwendung bei Menschen findet,
**dadurch gekennzeichnet,**
**dass** die Flavonoide nicht isoliert, sondern in einem speziellen Pflanzenextrakt enthalten sind, welche die Biowirksamkeit der Flavonoide erhöht.

5. Mischungen mit Polyphenolen und Omega-3 und -6 Fettsäuren, welche in jeder Form medizinische, therapeutische, präventive, nahrungsergänzende und kosmetische Anwendung bei Menschen findet,
**dadurch gekennzeichnet,**
**dass** die Polyphenole nicht isoliert, sondern in einem speziellen Pflanzenextrakt enthalten sind, welche die Biowirksamkeit der Polyphenole erhöht.

6. Mischungen mit sekundären Pflanzenstoffen und Omega-3 und -6 Fettsäuren, welche in jeder Form medizinische, therapeutische, präventive, nahrungsergänzende und kosmetische Anwendung bei Menschen findet,
**dadurch gekennzeichnet,**
**dass** die sekundären Pflanzenstoffe nicht isoliert, sondern in einem speziellen Pflanzenextrakt enthalten sind, welche die Biowirksamkeit der sekundären Pflanzenstoffe erhöht.
